(19) **Europäisches Patentamt**
**European Patent Office**
**Office européen des brevets**

(11) **EP 1 804 172 B1**

(12) **EUROPEAN PATENT SPECIFICATION**

(45) Date of publication and mention of the grant of the patent:
**11.08.2021 Bulletin 2021/32**

(51) Int Cl.:
**C12Q 1/686** *(2018.01)* **G06F 17/10** *(2006.01)*

(21) Application number: **06026168.2**

(22) Date of filing: **18.12.2006**

(54) **PCR elbow determination using curvature analysis of a double sigmoid**

Bestimmung des Wendepunktes einer PCR-Kurve mittels Krümmungsanalyse einer Doppelsigmoidfunktion

Procédé de détermination du point d'inflexion d'une courbe PCR par analyse de courbature d'une fonction double sigmoïde

(84) Designated Contracting States:
**AT BE BG CH CY CZ DE DK EE ES FI FR GB GR HU IE IS IT LI LT LU LV MC NL PL PT RO SE SI SK TR**

(30) Priority: **20.12.2005 US 316315**
**06.02.2006 US 349550**
**19.07.2006 US 458644**
**19.09.2006 US 533291**

(43) Date of publication of application:
**04.07.2007 Bulletin 2007/27**

(73) Proprietors:
• **Roche Diagnostics GmbH**
**68305 Mannheim (DE)**
Designated Contracting States:
**DE**
• **F. Hoffmann-La Roche AG**
**4070 Basel (CH)**
Designated Contracting States:
**AT BE BG CH CY CZ DK EE ES FI FR GB GR HU IE IS IT LI LT LU LV MC NL PL PT RO SE SI SK TR**

(72) Inventors:
• **Kurnik, Ronald T.**
**Foster City, CA 94404 (US)**

• **Wang, Jianmei**
**Mountain View, CA 94041 (US)**

(56) References cited:
**EP-A1- 1 647 910 US-A1- 2003 148 302**

• **TICHOPAD A ET AL: "Improving quantitative real-time RT-PCR reproducibility by boosting primer-linked amplification efficiency", BIOTECHNOLOGY LETTERS, SPRINGER NETHERLANDS, NL, vol. 24, no. 24, December 2002 (2002-12), pages 2053-2056, XP002309015, ISSN: 0141-5492**
• **ZHAO SHENG ET AL: "Comprehensive algorithm for quantitative real-time polymerase chain reaction", JOURNAL OF COMPUTATIONAL BIOLOGY, MARY ANN LIEBERT INC, US, vol. 12, no. 8, 1 October 2005 (2005-10-01), pages 1047-1064, XP002380886, ISSN: 1066-5277, DOI: 10.1089/CMB.2005.12.1047**
• **REBRIKOV D V ET AL: "Real-time PCR: A review of approaches to data analysis", APPLIED BIOCHEMISTRY AND MICROBIOLOGY, vol. 42, no. 5, September 2006 (2006-09), pages 455-463, XP019389693, ISSN: 1608-3024, DOI: 10.1134/S0003683806050024**
• **None**

**Description**

BACKGROUND OF THE INVENTION

The present invention relates generally to systems and methods for determining characteristic cycle threshold (Ct) or elbow values in PCR amplification curves, or elbow values in other growth curves.

The Polymerase Chain Reaction (PCR) is an *in vitro* method for enzymatically synthesizing or amplifying defined nucleic acid sequences. The reaction typically uses two oligonucleotide primers that hybridize to opposite strands and flank a template or target DNA sequence that is to be amplified. Elongation of the primers is catalyzed by a heat-stable DNA polymerase. A repetitive series of cycles involving template denaturation, primer annealing, and extension of the annealed primers by the polymerase results in an exponential accumulation of a specific DNA fragment. Fluorescent probes or markers are typically used in the process to facilitate detection and quantification of the amplification process.

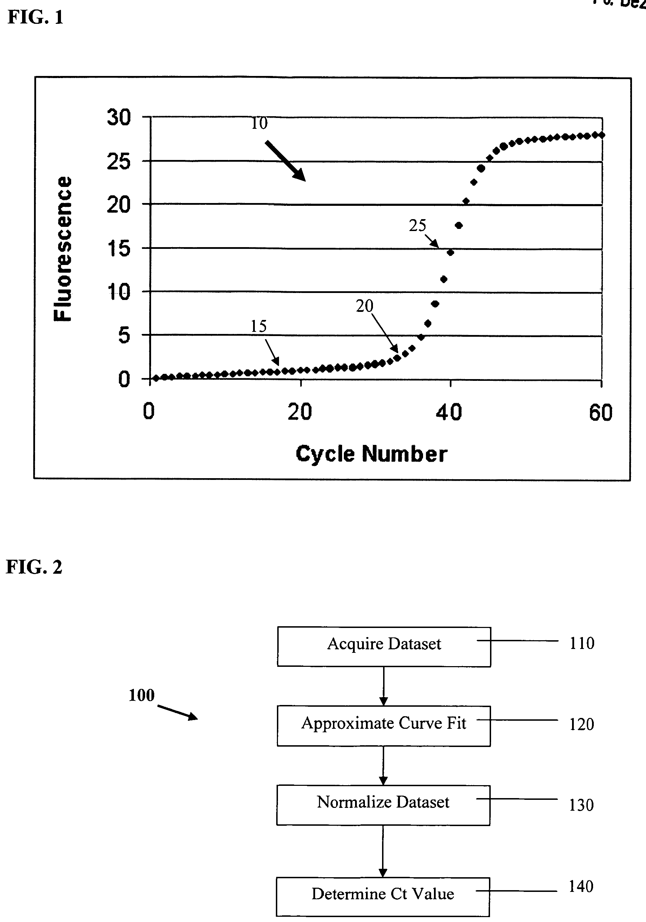

A typical real-time PCR curve is shown in FIG. 1, where fluorescence intensity values are plotted vs. cycle number for a typical PCR process. In this case, the formation of PCR products is monitored in each cycle of the PCR process. The amplification is usually measured in thermocyclers which include components and devices for measuring fluorescence signals during the amplification reaction. An example of such a thermocycler is the Roche Diagnostics LightCycler (Cat. No. 20110468). The amplification products are, for example, detected by means of fluorescent labelled hybridization probes which only emit fluorescence signals when they are bound to the target nucleic acid or in certain cases also by means of fluorescent dyes that bind to double-stranded DNA.

For a typical PCR curve, identifying a transition point at the end of the baseline region, which is referred to commonly as the elbow value or cycle threshold (Ct) value, is extremely useful for understanding characteristics of the PCR amplification process. The Ct value may be used as a measure of efficiency of the PCR process. For example, typically a defined signal threshold is determined for all reactions to be analyzed and the number of cycles (Ct) required to reach this threshold value is determined for the target nucleic acid as well as for reference nucleic acids such as a standard or housekeeping gene. The absolute or relative copy numbers of the target molecule can be determined on the basis of the Ct values obtained for the target nucleic acid and the reference nucleic acid (Gibson et al., Genome Research 6:995-1001; Bieche et al., Cancer Research 59:2759-2765, 1999; WO 97/46707; WO 97/46712; WO 97/46714). The elbow value in region 20 at the end of the baseline region 15 in FIG. 1 would be in the region of cycle number 30.

US 2003/148302 discloses a method for processing and evaluating real-time PCR data comprising identifying an exponential amplification region, determining upper and lower bounds, identifying a baseline to estimate and compensate for data noise, and determining the threshold cycle.

Tichopad et al., Biotechnology Letters, 2002, 24:2053-2056 discloses a four-parametric sigmoid model for mathematically approximating real-time PCR data.

EP 1647910 discloses a mathematical model for the detection of a specific nucleic acid within a sample comprising determining a deviation from a linear curve using a quasi linear regression.

The elbow value in a PCR curve can be determined using several existing methods. For example, various current methods determine the actual value of the elbow as the value where the fluorescence reaches a predetermined level called the AFL (arbitrary fluorescence value). Other current methods might use the cycle number where the second derivative of fluorescence vs. cycle number reaches a maximum. All of these methods have drawbacks. For example, some methods are very sensitive to outlier (noisy) data, and the AFL value approach does not work well for data sets with high baselines. Traditional methods to determine the baseline stop (or end of the baseline) for the growth curve shown in FIG. 1 may not work satisfactorily, especially in a high titer situation. Furthermore, these algorithms typically have many parameters (e.g., 50 or more) that are poorly defined, linearly dependent, and often very difficult, if not impossible, to optimize.

Therefore it is desirable to provide systems and methods for determining the elbow value in curves, such as sigmoid-type or growth curves, and PCR curves in particular, which overcome the above and other problems.

BRIEF SUMMARY OF THE INVENTION

Disclosed herein are systems and methods for determining characteristic transition values such as elbow values in sigmoid or growth-type curves. In the present invention, these systems and methods are used for determining the cycle threshold (Ct) value in PCR amplification curves.

According to the present invention, a double sigmoid function with parameters determined by a Levenberg-Marquardt (LM) regression process is used to find an approximation to a curve that fits a PCR dataset. Once the parameters have been determined, the curve can be normalized using one or more of the determined parameters. After normalization, the normalized curve is processed to determine the curvature of the curve at some or all points along the curve, e.g., to produce a dataset or plot representing the curvature v. the cycle number. The cycle number at which the maximum curvature occurs corresponds to the Ct value. The Ct value is then returned and may be displayed or otherwise used for further processing.

In a first aspect of the invention a computer-implemented method of determining the cycle threshold (Ct) value of a growth curve for a kinetic Polymerase Chain Reaction (PCR) process is provided, comprising the steps of:

- receiving a dataset representing a PCR growth curve, said dataset including a plurality of data points each having a pair of coordinate values (x,y), where the x-axis represents cycle number and the y-axis fluorescence intensity;
- calculating an approximation function that fits the dataset by applying a regression process to determine parameters of the function and a curve approximating the dataset;
- normalizing the curve approximating the dataset using the determined parameters to produce a normalized curve; and
- processing the normalized curve to determine a point of maximum curvature, wherein the point of maximum curvature represents the cycle threshold (Ct) value of the PCR growth curve;
  wherein
- the approximation function is a double sigmoid function of the form:

$$a + bx + \frac{c}{(1 + \exp^{-d(x-e)})(1 + \exp^{-f(x-g)})}$$

- calculating includes iteratively determining the parameters a, b, c, d, e, f and g of the function by applying a Levenberg-Marquardt (LM) regression process; and
- processing includes determining the curvature at some or all points along the normalized curve.

In a second aspect of the invention a computer-readable medium including code for controlling a processor to determine the cycle threshold (Ct) value of a growth curve for a kinetic Polymerase Chain Reaction (PCR) process is provided, the code including instructions to:

- receive a dataset representing a PCR growth curve, said dataset including a plurality of data points each having a pair of coordinate values (x,y), where the x-axis represents cycle number and the y-axis fluorescence intensity;
- calculate an approximation function that fits the dataset by applying a regression process to determine parameters of the function and a curve approximating the dataset;
- normalize the curve approximating the dataset using the determined parameters to produce a normalized curve; and
- process the normalized curve to determine a point of maximum curvature, wherein the point of maximum curvature represents the the cycle threshold (Ct) value of the PCR growth curve
  wherein
- the approximation function is a double sigmoid function of the form:

$$a + bx + \frac{c}{(1 + \exp^{-d(x-e)})(1 + \exp^{-f(x-g)})}$$

- calculating includes iteratively determining the parameters a, b, c, d, e, f and g of the function by applying a Levenberg-Marquardt (LM) regression process; and
- processing includes determining the curvature at some or all points along the normalized curve.

In yet another aspect of the invention a kinetic Polymerase Chain Reaction (PCR) system is provided, comprising:

- a kinetic PCR analysis module adapted to generate a PCR dataset representing a kinetic PCR amplification curve, said dataset including a plurality of data points, each having a pair of coordinate values (x,y), where the x-axis represents cycle number and the y-axis fluorescence intensity, wherein said dataset includes data points in a region of interest which includes a cycle threshold (Ct) value; and
- an intelligence module adapted to process the PCR dataset to determine the Ct value, by:
- calculating an approximation function that fits the dataset by applying a regression process to determine parameters of the function and a curve approximating the dataset;
- normalizing the curve approximating the dataset using the determined parameters to produce a normalized curve; and
- processing the normalized curve to determine a point of maximum curvature, wherein the point of maximum curvature represents the cycle threshold (Ct) value of the PCR growth curve

wherein
- the approximation function is a double sigmoid function of the form:

$$a + bx + \frac{c}{(1 + \exp^{-d(x-e)})(1 + \exp^{-f(x-g)})} ;$$

- calculating includes iteratively determining the parameters a, b, c, d, e, f and g of the function by applying a Levenberg-Marquardt (LM) regression process; and
- processing includes determining the curvature at some or all points along the normalized curve.

Reference to the remaining portions of the specification, including the drawings and claims, will realize other features and advantages of the present invention. Further features and advantages of the present invention, as well as the structure and operation of various embodiments of the present invention, are described in detail below with respect to the accompanying drawings. In the drawings, like reference numbers indicate identical or functionally similar elements.

BRIEF DESCRIPTION OF THE DRAWINGS

FIG. 1 illustrates an example of a typical PCR growth curve, plotted as fluorescence intensity vs. cycle number.

FIG. 2 shows a process flow for determining the end of a baseline region of a growth curve, or Ct value of a PCR curve.

FIG. 3 illustrates a detailed process flow for a spike identification and replacement process according to one embodiment of the present invention.

FIG. 4 illustrates a decomposition of the double sigmoid equation including parameters (a)-(g).

FIG. 5 shows the influence of parameter (d) on the curve and the position of (e), the x value of the inflexion point. All curves in FIG. 5 have the same parameter values except for parameter (d).

FIG. 6 shows an example of the three curve shapes for the different parameter sets.

FIG. 7 illustrates a process for determining the value of double sigmoid equation parameters (e) and (g) according to one aspect.

FIG. 8 illustrates a process flow of a Levenberg-Marquardt regression process for an initial set of parameters.

FIG. 9 illustrates a more detailed process flow for determining the elbow value for a PCR process according to one embodiment.

FIG. 10a shows a typical growth curve that was fit to experimental data using a double sigmoid, and

FIG. 10b shows a plot of a the curvature of the double sigmoid curve of FIG. 10a.

FIG. 11 shows a circle superimposed in the growth curve in FIG. 10a tangential to the point of maximum curvature.

FIG. 12a shows an example of a data set for a growth curve.

FIG. 12b shows a plot of the data set of FIG. 12a.

FIG. 13 shows a double sigmoid fit to the data set of FIG. 12.

FIG. 14 shows the data set (and double sigmoid fit) of FIG. 12 (FIG. 13) after normalization using the baseline subtraction method of equation (6).

FIG. 15 shows a plot of the curvature vs. cycle number for the normalized data set of FIG. 14.

FIG. 16 shows a superposition of a circle with the maximum radius of curvature and the normalized data set of FIG. 14.

FIG. 17 shows an example of a "slow-grower" data set.

FIG. 18 shows the data set of FIG. 17 and a double sigmoid fit after normalization using the baseline subtraction method of equation (6).

FIG. 19 shows a plot of the curvature vs. cycle number for the normalized data set of FIG. 18.

FIG 20 shows a plot of a set of PCR growth curves, including replicate runs and negative samples.

FIG. 21 shows a general block diagram depicting the relation between the software and hardware resources.

DETAILED DESCRIPTION OF THE INVENTION

Disclosed herein are systems and methods for determining a transition value in a sigmoid or growth curve, such as, as in the present invention, the end of the baseline region or the elbow value or Ct value of a PCR amplification curve. A double sigmoid function with parameters determined by a Levenberg-Marquardt (LM) regression process is used to find an approximation to the curve. Once the parameters have been determined, the curve is normalized using one or more of the determined parameters. After normalization, the normalized curve is processed to determine the curvature of the curve at some or all points along the curve, e.g., to produce a dataset or plot representing the curvature vs. the cycle number. The cycle number at which the maximum curvature occurs corresponds to the Ct value. The Ct value is then returned and may be displayed or otherwise used for further processing.

One example of a growth or amplification curve 10 in the context of a PCR process is shown in FIG. 1. As shown, the curve 10 includes a lag phase region 15, and an exponential phase region 25. Lag phase region 15 is commonly referred to as the baseline or baseline region. Such a curve 10 includes a transitionary region of interest 20 linking the lag phase and the exponential phase regions. Region 20 is commonly referred to as the elbow or elbow region. The elbow region typically defines an end to the baseline and a transition in the growth or amplification rate of the underlying process. Identifying a specific transition point in region 20 can be useful for analyzing the behavior of the underlying process. In a typical PCR curve, identifying a transition point referred to as the elbow value or cycle threshold (Ct) value is useful for understanding efficiency characteristics of the PCR process.

Other processes that may provide similar sigmoid or growth curves include bacterial processes, enzymatic processes and binding processes. In bacterial growth curves, for example, the transition point of interest has been referred to as the time in lag phase, $\theta$. Other specific processes that produce data curves that may be analyzed according to the present invention include strand displacement amplification (SDA) processes, nucleic acid sequence-based amplification (NASBA) processes and transcription mediated amplification (TMA) processes. Examples of SDA and NASBA processes and data curves can be found in Wang, Sha-Sha, et al., "Homogeneous Real-Time Detection of Single-Nucleotide Polymorphisms by Strand Displacement Amp lification on the BD ProbeTec ET System", Clin Chem 2003 49(10):1599, and Weusten, Jos J.A.M., et al., "Principles of Quantitation of Viral Loads Using Nucleic Acid Sequence-Based Amplification in Combination With Homogeneous Detection Using Molecular Beacons", Nucleic Acids Research, 2002 30(6):26, respectively. Thus, although the remainder of this document will discuss the disclosed systems and methods in terms of their applicability to PCR curves, according to the present invention, it should be appreciated that they could also be applied to data curves related to other processes, but these other applications are not part of the present invention.

As shown in FIG. 1, data for a typical PCR growth curve can be represented in a two-dimensional coordinate system, for example, with PCR cycle number defining the x-axis and an indicator of accumulated polynucleotide growth defining the y-axis. Typically, as shown in FIG. 1, the indicator of accumulated growth is a fluorescence intensity value as the use of fluorescent markers is perhaps the most widely used labeling scheme. However, it should be understood that, in examples not part of the present invention, other indicators may be used depending on the particular labeling and/or detection scheme used. Examples of other useful indicators of accumulated signal growth include luminescence intensity, chemiluminescence intensity, bioluminescence intensity, phosphorescence intensity, charge transfer, voltage, current, power, energy, temperature, viscosity, light scatter, radioactive intensity, reflectivity, transmittance and absorbance. The definition of cycle can also include time, process cycles, unit operation cycles and reproductive cycles.

**General Process Overview**

According to the present invention, one embodiment of a process 100 for determining the elbow value or Ct value of a kinetic PCR amplification curve can be described briefly with reference to FIG. 2. In step 110, an experimental data set representing the curve is received or otherwise acquired. An example of a plotted PCR data set is shown in FIG. 1, where the y-axis and x-axis represent fluorescence intensity and cycle number, respectively, for a PCR curve. In certain aspects, the data set should include data that is continuous and equally spaced along an axis.

In an exemplary embodiment of the present invention, the method may be implemented by using conventional personal computer systems including, but not limited to, an input device to input a data set, such as a keyboard, mouse, and the like; a display device to represent a specific point of interest in a region of a curve, such as a monitor; a processing device necessary to carry out each step in the method, such as a CPU; a network interface such as a modem, a data storage device to store the data set, a computer code running on the processor and the like. Furthermore, the method may also be implemented in a PCR device.

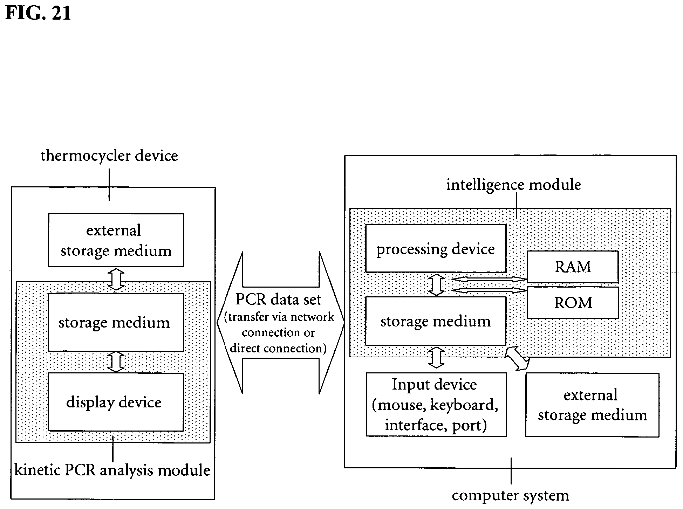

A system according to the invention is displayed in FIG. 21. This figure shows a general block diagram explaining the relation between the software and hardware resources. The system comprises a kinetic PCR analysis module which may be located in a thermocycler device and an intelligence module which is part of the computer system. The data sets (PCR data sets) are transferred from the analysis module to the intelligence module or vice versa via a network connection or a direct connection. The data sets are processed according to the method as displayed in Fig. 2 by computer code running on the processor and being stored on the storage device of the intelligence module and after processing transferred back to the storage device of the analysis module, where the modified data may be displayed on a displaying device. In a particular embodiment the intelligence module may also be implemented in the PCR data acquiring device.

In the case where process 100 is implemented in an intelligence module (e.g., processor executing instructions) resident in a PCR data acquiring device such as a thermocycler, the data set may be provided to the intelligence module in real time as the data is being collected, or it may be stored in a memory unit or buffer and provided to the intelligence module after the experiment has been completed. Similarly, the data set may be provided to a separate system such as a desktop computer system or other computer system, via a network connection (e.g., LAN, VPN, intranet, Internet, etc.) or direct connection (e.g., USB or other direct wired or wireless connection) to the acquiring device, or provided on a portable medium such as a CD, DVD, floppy disk or the like. In certain aspects, the data set includes data points having a pair of coordinate values (or a 2-dimensional vector). For PCR data, the pair of coordinate values typically represents the cycle number and the fluorescence intensity value. After the data set has been received or acquired in step 110, the data set may be analyzed to determine the end of the baseline region.

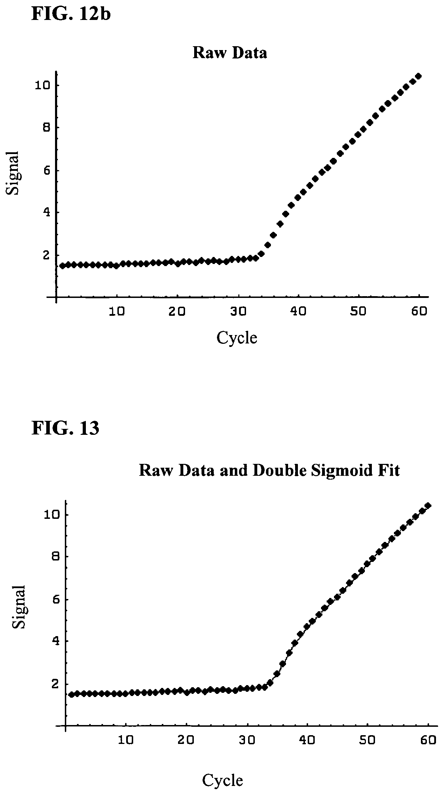

In step 120, an approximation of the curve is calculated. During this step, a double sigmoid function with parameters determined by a Levenberg-Marquardt (LM) regression process is used to find an approximation of a curve representing the data set. The approximation is said to be "robust" as outlier or spike points have a minimal effect on the quality of the curve fit. FIG. 13, which will be discussed below, illustrates an example of a plot of a received data set and a robust approximation of the data set determined by using a Levenberg-Marquardt regression process to determine the parameters of a double sigmoid function according to the present invention.

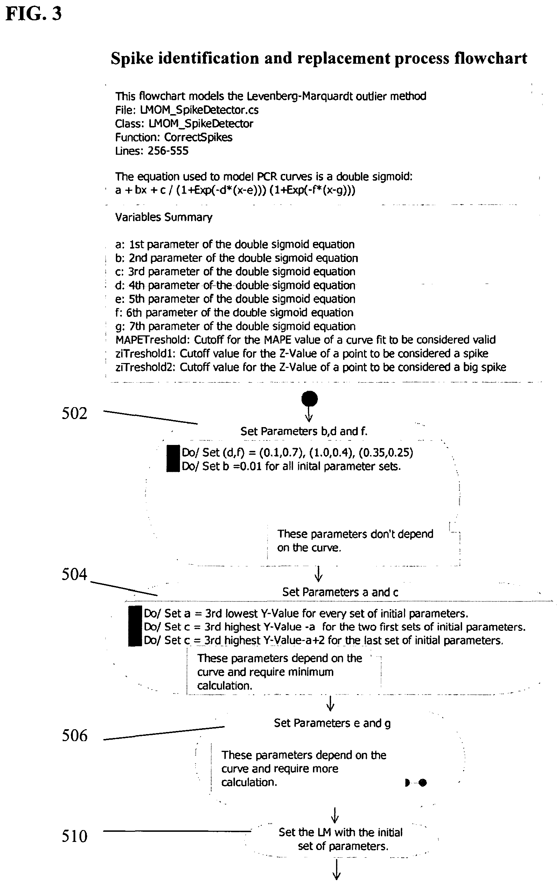

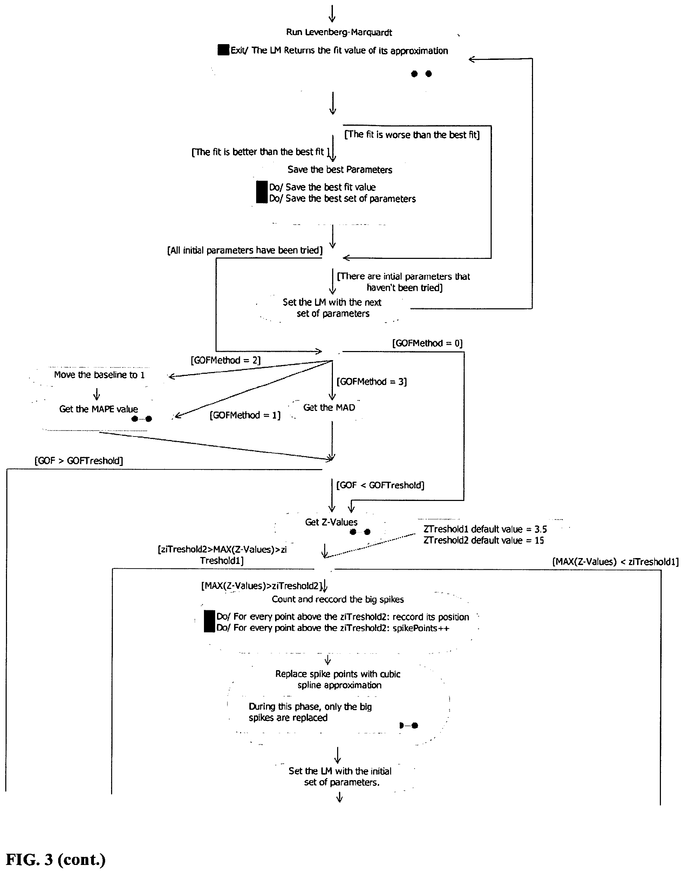

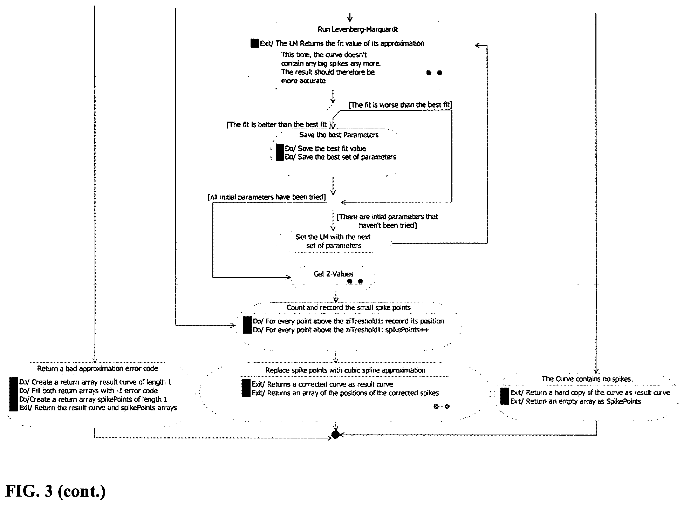

In certain aspects, outlier or spike points in the dataset are removed or replaced prior to processing the data set to determine the end of the baseline region. Spike removal may occur before or after the dataset is acquired in step 110. FIG. 3 illustrates the process flow for identifying and replacing spike points in datasets representing PCR or other growth curves.

In step 130, the parameters determined in step 120 are used to normalize the curve, e.g., to remove the baseline slope, as will be described in more detail below. Normalization in this manner allows for determining the Ct value without having to determine or specify the end of the baseline region of the curve or a baseline stop position. In step 140, the normalized curve is then processed to determine the Ct value as will be discussed in more detail below.

## LM Regression Process

Steps 502 through 524 of FIG. 3, as will be discussed below, illustrate a process flow for approximating the curve of a dataset and determining the parameters of a fit function (step 120). These parameters can be used in normalizing the curve, e.g., modifying or removing the baseline slope of the data set representing a sigmoid or growth type curve such as a PCR curve according to one embodiment of the present invention (step 130). Where the dataset has been processed to produce a modified dataset with removed or replaced spike points, the modified spikeless dataset may be processed according to steps 502 through 524 to identify the parameters of the fit function.

In the present invention, a Levenberg-Marquardt (LM) method is used to calculate a robust curve approximation of a data set. The LM method is a non-linear regression process; it is an iterative technique that minimizes the distance between a non-linear function and a data set. The process behaves like a combination of a steepest descent process and a Gauss-Newton process: when the current approximation doesn't fit well it behaves like the steepest descent process (slower but more reliable convergence), but as the current approximation becomes more accurate it will then behave like the Gauss-Newton process (faster but less reliable convergence). The LM regression method is widely used to solve non-linear regression problems.

In general, the LM regression method includes an algorithm that requires various inputs and provides output. In one aspect, the inputs include a data set to be processed, a function that is used to fit the data, and an initial guess for the parameters or variables of the function. The output includes a set of parameters for the function that minimizes the distance between the function and the data set.

According to the present invention, the fit function is a double sigmoid of the form:

$$f(x) = a + bx + \frac{c}{(1 + \exp^{-d(x-e)})(1 + \exp^{-f(x-g)})} . \qquad (1)$$

The choice of this equation as the fit function is based on its flexibility and its ability to fit the different curve shapes that a typical PCR curve or other growth curve may take.

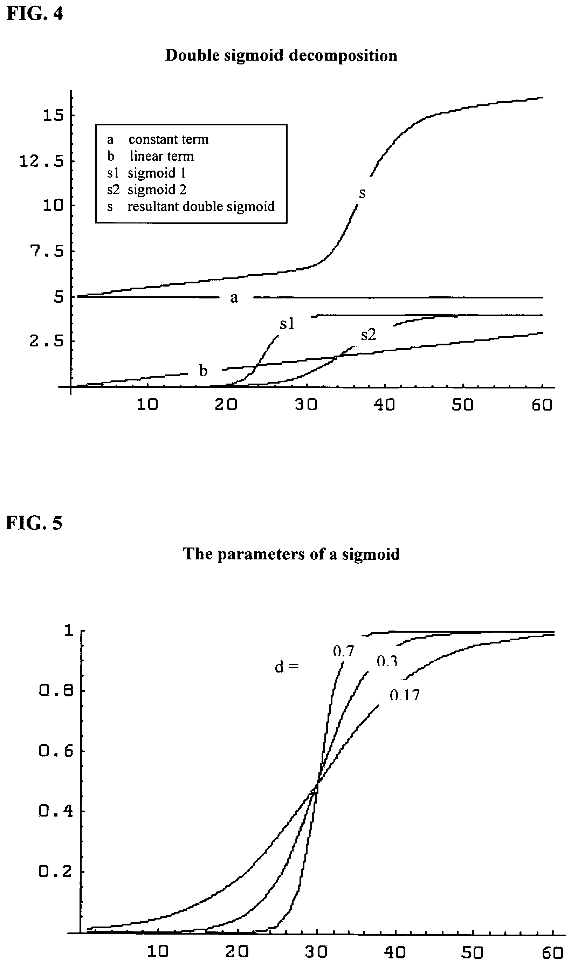

The double sigmoid equation (1) has 7 parameters: a, b, c, d, e, f and g. The equation can be decomposed into a sum of a constant, a slope and a double sigmoid. The double sigmoid itself is the multiplication of two sigmoids. FIG. 4 illustrates a decomposition of the double sigmoid equation (1). The parameters d, e, f and g determine the shape of the two sigmoids. To show their influence on the final curve, consider the single sigmoid:

$$\frac{1}{1 + \exp^{-d(x-e)}} , \qquad (2)$$

where the parameter d determines the "sharpness" of the curve and the parameter e determines the x-value of the inflexion point. FIG. 5 shows the influence of the parameter d on the curve and of the parameter e on the position of the x value of the inflexion point. Table 1, below, describes the influence of the parameters on the double sigmoid curve.

**Table 1: Double sigmoid parameters description**

| Parameter | Influence on the curve |
|---|---|
| a | Value of y at x = 0 |
| b | baseline and plateau slope |
| c | AFI of the curve |
| d | "sharpness" of the first sigmoid (See Figure 5) |
| e | position of the inflexion point of the first sigmoid (See Figure 5) |
| f | "sharpness" of the second sigmoid |
| g | position of the inflexion point of the second sigmoid |

In one aspect, the "sharpness" parameters d and f of the double sigmoid equation should be constrained in order to prevent the curve from taking unrealistic shapes. Therefore, in one aspect, any iterations where d<-1 or d > 1.1 or where f<-1 or f > 1.1 is considered unsuccessful. In other aspects, different constraints on parameters d and f may be used.

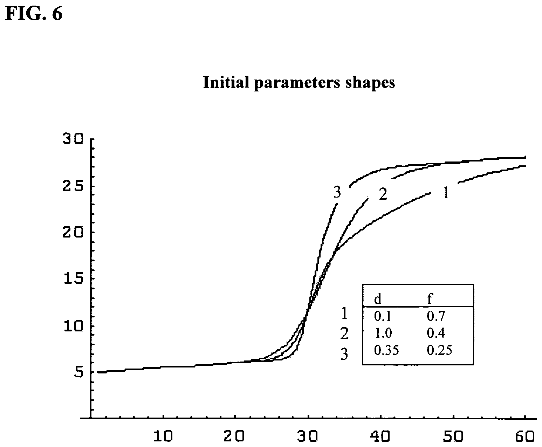

Because the Levenberg-Marquardt algorithm is an iterative algorithm, an initial guess for the parameters of the function to fit is typically needed. The better the initial guess, the better the approximation will be and the less likely it is that the algorithm will converge towards a local minimum. Due to the complexity of the double sigmoid function and the various shapes of PCR curves or other growth curves, one initial guess for every parameter may not be sufficient to prevent the algorithm from sometimes converging towards local minima. Therefore, in one aspect, multiple (e.g., three or more) sets of initial parameters are input and the best result is kept. In one aspect, most of the parameters are held constant across the multiple sets of parameters used; only parameters c, d and f may be different for each of the multiple parameter sets. FIG. 6 shows an example of the three curve shapes for the different parameter sets. The choice of these three sets of parameters is indicative of three possible different shapes of curves representing PCR data. It should be understood that more than three sets of parameters may be processed and the best result kept.

As shown in FIG. 3, the initial input parameters of the LM method are identified in step 510. These parameters may be input by an operator or calculated. According to one aspect, the parameters are determined or set according to steps 502, 504 and 506 as discussed below.

Calculation of initial parameter (a):

The parameter (a) is the height of the baseline; its value is the same for all sets of initial parameters. In one aspect, in step 504 the parameter (a) is assigned the 3rd lowest y-axis value, e.g., fluorescence value, from the data set. This provides for a robust calculation. In other aspects, of course, the parameter (a) may be assigned any other fluorescence value as desired such as the lowest y-axis value, second lowest value, etc.

Calculation of initial parameter (b):

The parameter (b) is the slope of the baseline and plateau. Its value is the same for all sets of initial parameters. In one aspect, in step 502 a static value of 0.01 is assigned to (b) as ideally there shouldn't be any slope. In other aspects, the parameter (b) may be assigned a different value, for example, a value ranging from 0 to about 0.5.

Calculation of initial parameter (c):

The parameter (c) represents the height of the plateau minus the height of the baseline, which is denoted as the absolute fluorescence increase, or AFI. In one aspect, for the first set of parameters, c = AFI + 2, whereas for the last two parameters, c = AFI. This is shown in FIG. 6, where for the last two sets of parameters, c = AFI. For the first set of parameters, c = AFI+2. This change is due to the shape of the curve modeled by the first set of parameters, which doesn't have a plateau.

Calculation of parameters (d) and (f):

The parameters (d) and (f) define the sharpness of the two sigmoids. As there is no way of giving an approximation based on the curve for these parameters, in one aspect three static representative values are used in step 502. It should be understood that other static or non-static values may be used for parameters (d) and/or (f). These pairs model the most common shapes on PCR curves encountered.

Table 2, below, shows the values of (d) and (f) for the different sets of parameters as shown in FIG. 6.

Table 2: Values of parameters d and f

| Parameter set number | Value of d | Value of f |
|---|---|---|
| 1 | 0.1 | 0.7 |
| 2 | 1.0 | 0.4 |
| 3 | 0.35 | 0.25 |

Calculation of parameters (e) and (g):

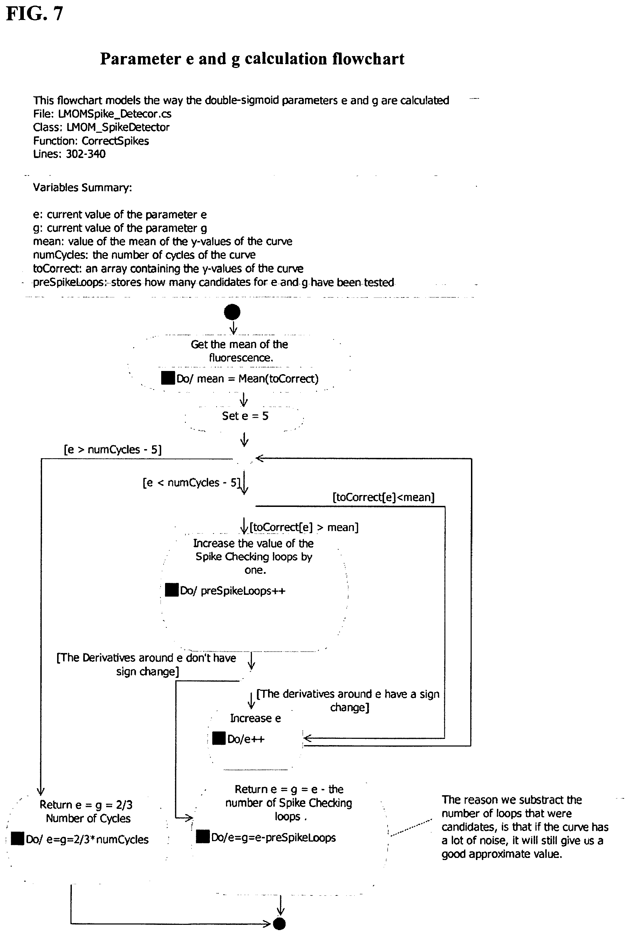

In step 506, the parameters (e) and (g) are determined. The parameters (e) and (g) define the inflexion points of the two sigmoids. In one aspect, they both take the same value across all the initial parameter sets. Parameters (e) and (g) may have the same or different values. To find an approximation, in one aspect, the x-value of the first point above the mean of the intensity, e.g., fluorescence, (which isn't a spike) is used. A process for determining the value of (e) and (g) according to this aspect is shown in FIG. 7 and discussed below.

With reference to FIG. 7, initially, the mean of the curve (e.g., fluorescence intensity) is determined. Next, the first data point above the mean is identified. It is then determined whether:

a. that point does not lie near the beginning, e.g., within the first 5 cycles, of the curve;

b. that point does not lie near the end, e.g., within the 5 last cycles, of the curve; and

c. the derivatives around the point (e.g., in a radius of 2 points around it) do not show any change of sign. If they do, the point is likely to be a spike and should therefore be rejected.

Table 3, below, shows examples of initial parameter values as used in FIG. 6 according to one aspect.

Table 3: Initial parameters values:

| Initial parameter set number | 1 | 2 | 3 |
|---|---|---|---|
| Value of a | 3rd lowest fluorescence value | 3rd lowest fluorescence value | 3rd lowest fluorescence value |
| Value of b | 0.01 | 0.01 | 0.01 |
| Value of c | 3rd highest fluorescence value - a + 2 | 3rd highest fluorescence value - a | 3rd highest fluorescence value - a |
| Value of d | 0.1 | 1.0 | 0.35 |
| Value of e | X of the first non-spiky point above the mean of the fluorescence | X of the first non-spiky point above the mean of the fluorescence | X of the first non-spiky point above the mean of the fluorescence |
| Value of f | 0.7 | 0.4 | 0.25 |
| Value of g | X of the first non-spiky point above the mean of the fluorescence | X of the first non-spiky point above the mean of the fluorescence | X of the first non-spiky point above the mean of the fluorescence |

Returning to FIG. 3, once all the parameters are set in step 510, a LM process 520 is executed using the input data set, function and parameters. Traditionally, the Levenberg-Marquardt method is used to solve non-linear least-square problems. The traditional LM method calculates a distance measure defined as the sum of the square of the errors between the curve approximation and the data set. However, when minimizing the sum of the squares, it gives outliers an important weight as their distance is larger than the distance of non-spiky data points, often resulting in inappropriate curves or less desirable curves. Therefore, according to one aspect of the present invention, the distance between the approximation and the data set is computed by minimizing the sum of absolute errors as this does not give as much weight to the outliers. In this aspect, the distance between the approximation and data is given by:

$$\text{distance} = \sum \left| y_{data} - y_{approximation} \right|. \qquad (3)$$

As above, in one aspect, each of the multiple (e.g., three) sets of initial parameters are input and processed and the best result is kept as shown in steps 522 and 524, where the best result is the parameter set that provides the smallest or minimum distance in equation (3). In one aspect, most of the parameters are held constant across the multiple sets of parameters; only c, d and f may be different for each set of parameters. It should be understood that any number of initial parameter sets may be used.

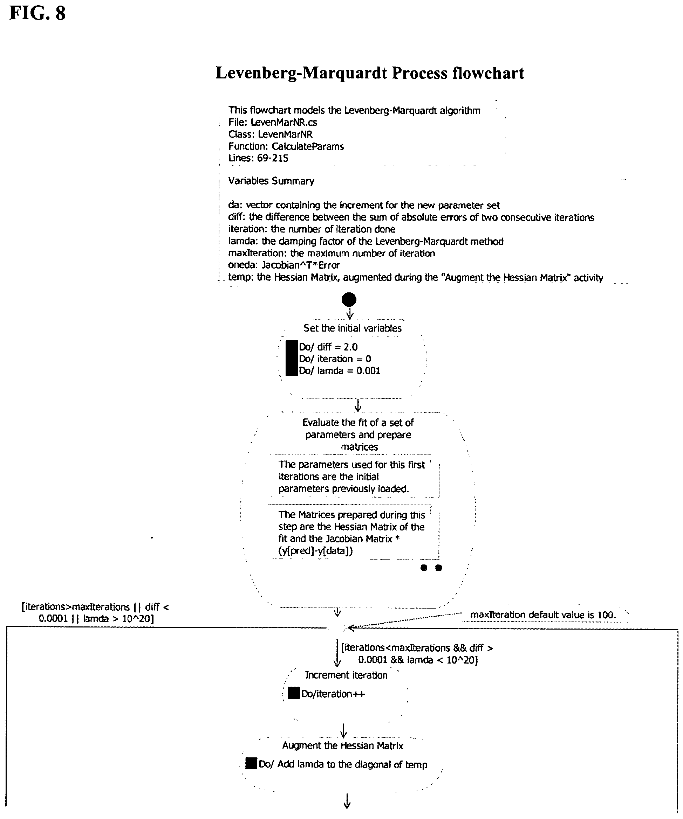

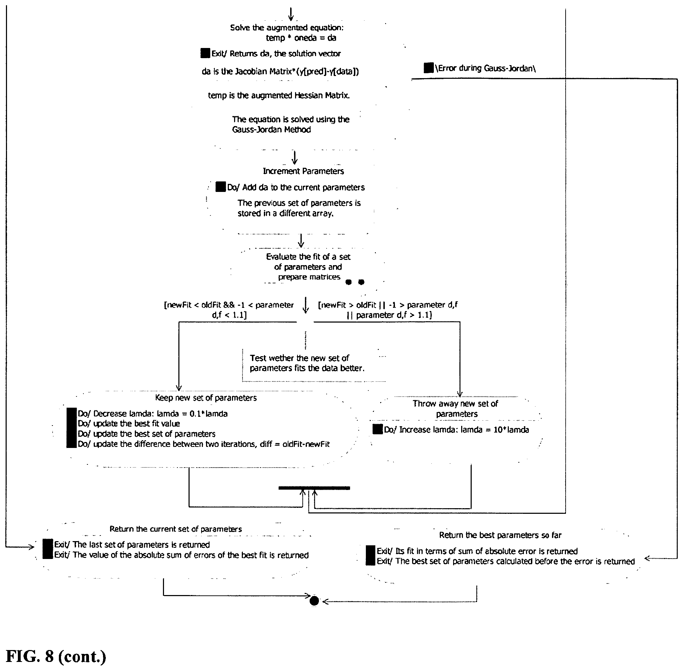

FIG. 8 illustrates a process flow of LM process 520 for a set of parameters according to the present invention. As explained above, the Levenberg-Marquardt method can behave either like a steepest descent process or like a Gauss-Newton process. Its behavior depends on a damping factor $\lambda$. The larger $\lambda$ is, the more the Levenberg-Marquardt algorithm will behave like the steepest descent process. On the other hand, the smaller $\lambda$ is, the more the Levenberg-Marquardt algorithm will behave like the Gauss-Newton process. In one aspect, $\lambda$ is initiated at 0.001. It should be appreciated that $\lambda$ may be initiated at any other value, such as from about 0.000001 to about 1.0.

As stated before, the Levenberg-Marquardt method is an iterative technique. According to one aspect, as shown in FIG. 8 the following is done during each iteration:

1. The Hessian Matrix (H) of the precedent approximation is calculated.
2. The transposed Jacobian Matrix ($J^T$) of the precedent approximation is calculated.
3. The distance vector (d) of the precedent approximation is calculated.
4. The Hessian Matrix diagonal is augmented by the current damping factor $\lambda$:

$$H_{aug} = H\lambda \qquad (4)$$

5. Solve the augmented equation:

$$H_{aug} x = J^T d \qquad\qquad (5)$$

6. The solution x of the augmented equation is added to the parameters of the function.

7. Calculate the distance between the new approximation and the curve.

8. If the distance with this new set of parameters is smaller than the distance with the previous set of parameters:

- The iteration is considered successful.

- Keep or store the new set of parameters.

- Decrease the damping factor $\lambda$, e.g., by a factor 10.

If the distance with this new set of parameters is larger than the distance with the previous set of parameters:

- The iteration is considered unsuccessful.
- Throw away the new set of parameters.
- Increase the damping factor $\lambda$, e.g., by a factor of 10.

In one aspect, the LM process of FIG. 8 iterates until one of the following criteria is achieved:

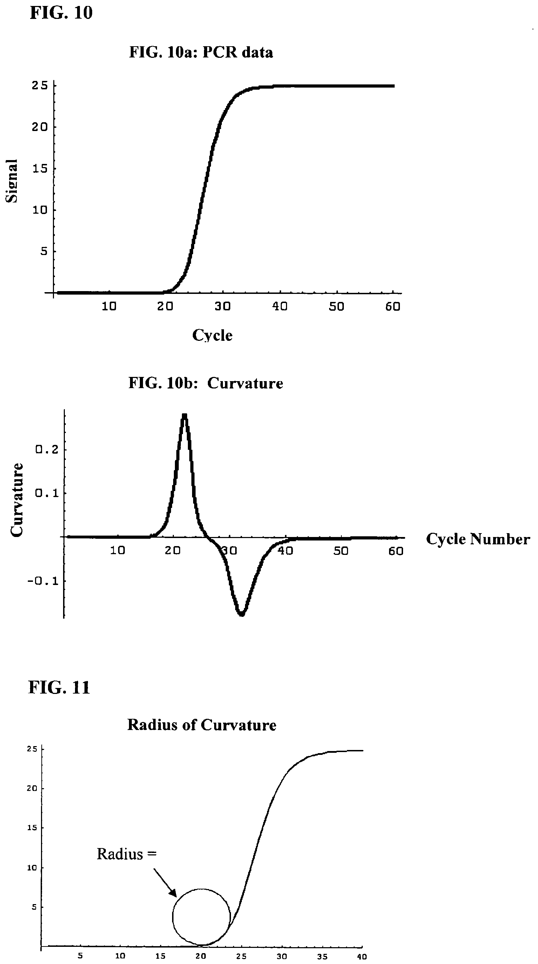

1. It has run for a specified number, N, of iterations. This first criterion prevents the algorithm from iterating indefinitely. For example, in one aspect as shown in FIG. 10, the default iteration value N is 100. 100 iterations should be plenty for the algorithm to converge if it can converge. In general, N can range from fewer than 10 to 100 or more.

2. The difference of the distances between two successful iterations is smaller than a threshold value. e.g., 0.0001. When the difference becomes very small, the desired precision has been achieved and continuing to iterate is pointless as the solution won't become significantly better.

3. The damping factor $\lambda$ exceeds a specified value, e.g., is larger than $10^{20}$. When $\lambda$ becomes very large, the algorithm won't converge any better than the current solution, therefore it is pointless to continue iterating. In general, the specified value can be significantly smaller or larger than $10^{20}$.

**Normalization**

After the parameters have been determined, the curve is normalized (step 130) using one or more of the determined parameters. For example, in one aspect, the curve may be normalized or adjusted to have zero baseline slope by subtracting out the linear growth portion of the curve. Mathematically, this is shown as:

$$dataNew(BLS) = data-(a+bx), \qquad\qquad (6)$$

where dataNew(BLS) is the normalized signal after baseline subtraction, e.g., the data set (data) with the linear growth or baseline slope subtracted off or removed. The values of parameters a and b are those values determined by using the LM equation to regress the curve, and x is the cycle number. Thus, for every data value along the x-axis, the constant a and the slope b times the x value is subtracted from the data to produce a data curve with a zero baseline slope. In certain aspects, spike points are removed from the dataset prior to applying the LM regression process to the dataset to determine normalization parameters.

In another aspect, the curve may be normalized or adjusted to have zero slope according to the following equation:

$$dataNew(BLSD) = (data-(a+bx))/a, \qquad\qquad (7a)$$

where dataNew(BLSD) is the normalized signal after baseline subtraction with division, e.g., the data set (data) with the linear growth or baseline slope subtracted off or removed and the result divided by a. The value of parameters a and b are those values determined by using the LM equation to regress the curve, and x is the cycle number. Thus, for every data value along the x-axis, the constant a and the slope b times the x value is subtracted from the data and the result divided by the value of parameter a to produce a data curve with a zero baseline slope. In one aspect, equation (7a) is valid for parameter "a" $\geq$ 1; in the case where parameter "a" < 1, then the following equation is used:

$$dataNew(BLSD) = data-(a+bx). \qquad (7b)$$

In certain aspects, spike points are removed from the dataset prior to applying the LM regression process to the dataset to determine normalization parameters.

In yet another aspect, the curve may be normalized or adjusted according to following equation:

$$dataNew(BLD) = data/a, \qquad (8a)$$

where dataNew(BLD) is the normalized signal after baseline division, e.g., the data set (data) divided by parameter a. The values are the parameters a and b are those values determined by using the LM equation to regress to curve, and x is the cycle number. In one aspect, equation (8a) is valid for parameter "a" $\geq$ 1; in the case where parameter "a" < 1, then the following equation is used:

$$dataNew(BLD) = data + (1-a). \qquad (8b)$$

In certain aspects, spike points are removed from the dataset prior to applying the LM regression process to the dataset to determine normalization parameters.

In yet another aspect, the curve may be normalized or adjusted according to following equation:

$$dataNew(PGT) = (data-(a+bx))/c, \qquad (9a)$$

where dataNew(PGT) is the normalized signal after baseline subtraction with division, e.g., the data set (data) with the linear growth or baseline slope subtracted off or removed and the result divided by c. The value of parameters a, b and c are those values determined by using the LM equation to regress the curve, and x is the cycle number. Thus, for every data value along the x-axis, the constant a and the slope b times the x value is subtracted from the data and the result divided by the value of parameter c to produce a data curve with a zero baseline slope. In one aspect, equation (9a) is valid for parameter "c" $\geq$ 1; in the case where parameter "c" < 1 and "c" $\geq$ 0, then the following equation is used:

$$dataNew(PGT) = data-(a+bx). \qquad (9b)$$

In certain aspects, spike points are removed from the dataset prior to applying the LM regression process to the dataset to determine normalization parameters.

One skilled in the art will appreciate that other normalization equations may be used to normalized and/or modify the baseline using the parameters as determined by the Levenberg-Marquardt regression process.

**Curvature Determination**

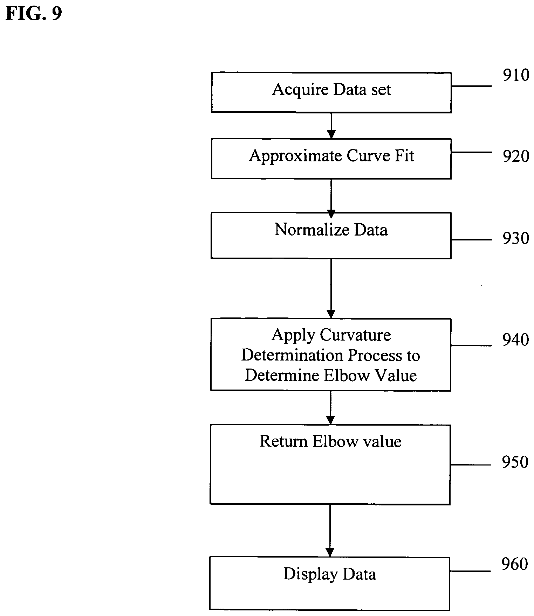

After the curve has been normalized using one of equations (6), (7), (8) or (9), or other normalization equation, the Ct value can be determined. In one embodiment, a curvature determination process or method is applied to the normalized curve as will be described with reference to FIG. 9, which shows a process flow for determining the elbow value or Ct value in a kinetic PCR curve. In step 910, the data set is acquired. In the case where the determination process is implemented in an intelligence module (e.g., processor executing instructions) resident in a PCR data acquiring device such as a thermocycler, the data set may be provided to the intelligence module in real time as the data is being collected, or it may be stored in a memory unit or buffer and provided to the module after the experiment has been completed. Similarly, the data set may be provided to a separate system such as a desktop computer system via a network connection (e.g., LAN, VPN, intranet, Internet, etc.) or direct connection (e.g., USB or other direct wired or wireless connection) to the acquiring device, or provided on a portable medium such as a CD, DVD, floppy disk or the like.

After a data set has been received or acquired, in step 920 an approximation to the curve is determined. During this step, a double sigmoid function with parameters determined by a Levenberg Marquardt regression process is used to find an approximation of a curve representing the dataset. Additionally, spike points may be removed from the dataset prior to step 920 as described with reference to FIG. 3. For example, the dataset acquired in step 910 can be a dataset with spikes already removed. In step 930, the curve is normalized. In certain aspects, the curve is normalized using one of equations (6), (7), (8) or (9) above. For example, the baseline may be set to zero slope using the parameters of the double sigmoid equation as determined in step with 920 to subtract off the baseline slope as per equation (6) above. In step 940, a process is applied to the normalized curve to determine the curvature at points along the normalized curve. A plot of the curvature v. cycle number may be returned and/or displayed. The point of maximum curvature corresponds to the elbow or Ct value. In step 950, the result is returned, for example to the system that performed the analysis, or to a separate system that requested the analysis. In step 960, Ct value is displayed. Additional data such as the entire data set or the curve approximation may also be displayed. Graphical displays may be rendered with a display device, such as a monitor screen or printer, coupled with the system that performed the analysis of FIG. 9, or data may be provided to a separate system for rendering on a display device.

To obtain the Ct value for this curve, the maximum curvature is determined. In one aspect, the curvature is determined for some or all points on the normalized curve. A plot of the curvature vs. cycle number may be displayed. The curvature of a curve is given by the equation, below:

$$kappa(x) = \frac{\left(\dfrac{d^2 y}{dx^2}\right)}{\left[1 + \left(\dfrac{dy}{dx}\right)^2\right]^{3/2}} \ . \tag{10}$$

Consider a circle of radius a, given by the equation below:

$$y(x) = \sqrt{a^2 - x^2} \tag{11}$$

The curvature of equation (11) is kappa(x) = -(1/a). Thus, the radius of curvature is equal to the negative inverse of the curvature. Since the radius of a circle is constant, its curvature is given by - (1/a). Now consider FIG. 10b, which is a plot of the curvature of the fit of the PCR data set of FIG. 10a. The Ct value can be considered to occur at the position of maximum curvature, which occurs at cycle number Ct = 21.84. This Ct value compares favorably to the PCR growth curve shown in FIG. 10a.

The radius of curvature at the maximum curvature (corresponding to a Ct value of 21.84) is: radius = 1/0.2818 = 3.55 cycles. A circle of this radius superimposed in the PCR growth curve in FIG. 10a is shown in FIG. 11. As FIG. 11 illustrates, a circle of radius corresponding to the maximum curvature represents the largest circle that can be superimposed at the start of the growth region of the PCR curve while remaining tangent to the curve. Curves with a small (maximum) radius of curvature will have steep growth curves while curves with a large (maximum) radius of curvature will have shallow growth curves. If the radius of curvature is extremely large, this is indicative of curves with no apparent signal.

The first and second derivatives of the double sigmoid of equation (1) that are needed in calculating the curvature are shown below.

**First Derivative**

$$\frac{dy}{dx} = b + \frac{ce^{-f(x-g)} f}{\left(1 + e^{-d(x-e)}\right)\left(1 + e^{-f(x-g)}\right)^2} + \frac{cde^{-d(x-e)}}{\left(1 + e^{-d(x-e)}\right)^2 \left(1 + e^{-f(x-g)}\right)} \tag{12}$$

**Equation (13): Second Derivative**

$$\frac{d^2y}{dx^2} = \frac{c\left(\frac{2d^2e^{-2d(x-e)}}{\left(1+e^{-d(x-e)}\right)^3} - \frac{d^2e^{-d(x-e)}}{\left(1+e^{-d(x-e)}\right)^2}\right)}{1+e^{-f(x-g)}} + \frac{2cde^{-d(x-e)-f(x-g)}f}{\left(1+e^{-d(x-e)}\right)^2\left(1+e^{-f(x-g)}\right)^2} + \frac{c\left(\frac{2e^{-2f(x-g)}f^2}{\left(1+e^{-f(x-g)}\right)^3} - \frac{e^{-f(x-g)}f^2}{\left(1+e^{-f(x-g)}\right)^2}\right)}{1+e^{-d(x-e)}}$$

## Examples

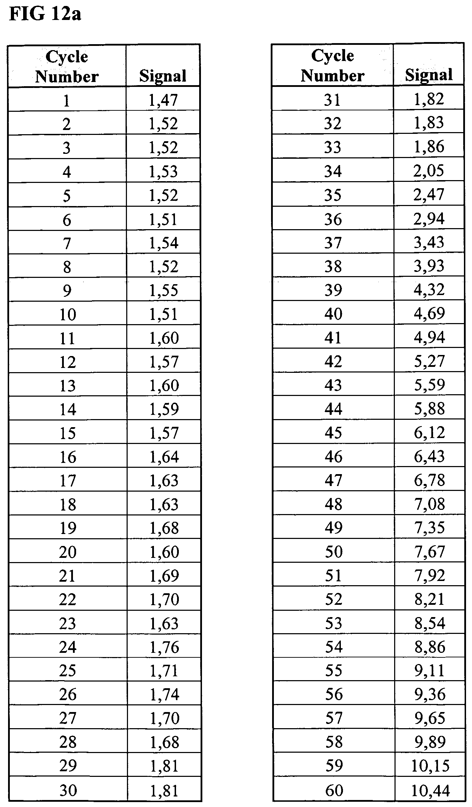

FIG. 12a shows an example of raw data for a growth curve. Applying the double sigmoid/LM method to the raw data plot shown in FIG. 12b gives values of the seven parameters in equation (1) as shown in Table 4 below:

Table 4

| a | 1.4707 |
|---|--------|
| b | 0.0093 |
| c | 10.9421 |
| d | 0.7858 |
| e | 35.9089 |
| f | 0.1081 |
| g | 49.1868 |

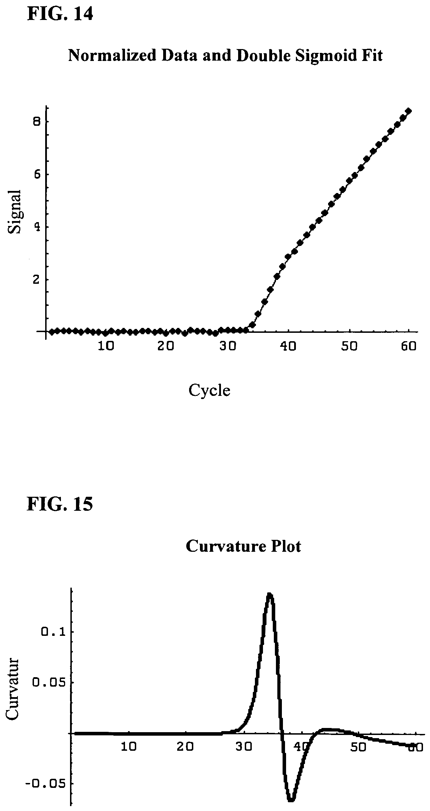

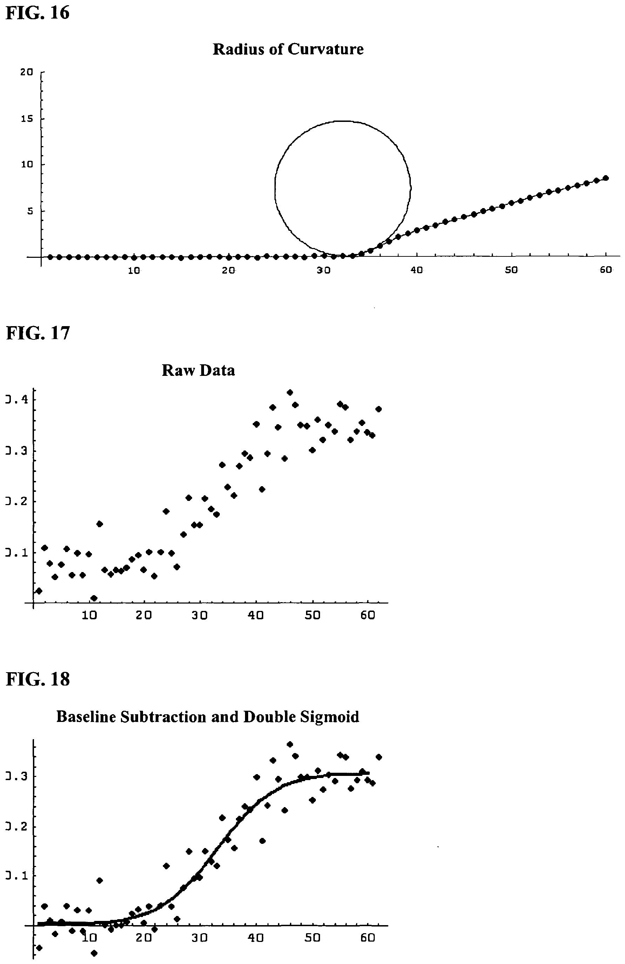

The double sigmoid fit to the data shown in FIG. 12 is shown in FIG. 13, indicating a very accurate assessment of the data points. These data were then normalized according to equation (6) (baseline subtraction) to yield the graph shown in FIG. 14. The solid line shown in FIG. 14 is the double sigmoid/LM application of equation (1) to the data set, which has been normalized according to equation (6). FIG. 15 shows a plot of the curvature v. cycle number for the normalized curve of FIG. 14. The maximum in the curvature occurs at cycle number 34.42 at a curvature of 0.1378. Thus, Ct = 34.42 based on the cycle number at maximum curvature, and the radius of curvature = 1/0.1378 = 7.25. A superposition of a circle with this radius of curvature and the normalized data set is shown in FIG. 16.

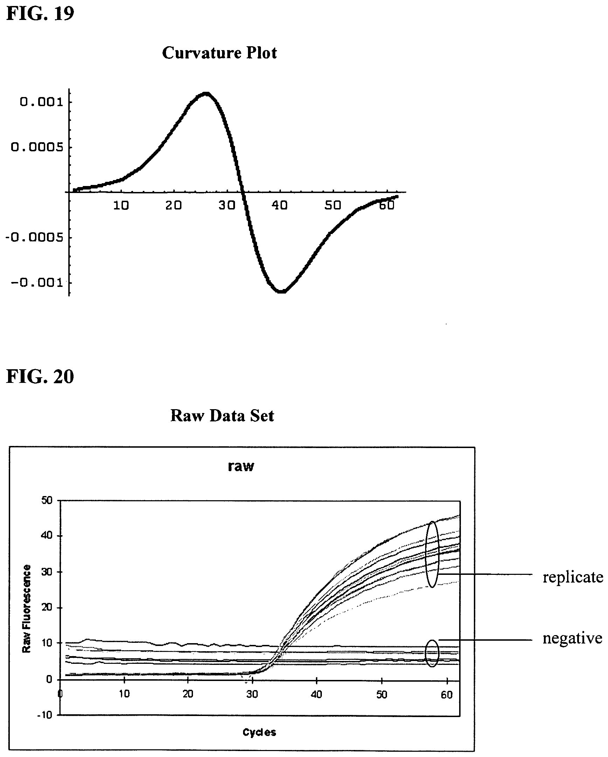

An example of a "slow-grower" data set is shown in FIG. 17. A double sigmoid fit to this data set and normalization using baseline subtraction, equation (6), gives the fit result shown in FIG. 18. The corresponding curvature plot is shown in FIG. 19. The maximum curvature occurs at cycle number 25.90, with a curvature = 0.00109274, corresponding to a radius of curvature = 915. This large radius of curvature communicates that this is a slow grower data set.

As another example, consider the set of PCR growth curves shown in FIG. 20. A comparison of the Ct values obtained using an existing method ("Threshold") vs. using the curvature method following baseline subtraction with division (BLSD - equation (7)) is shown in Table 5 below.

**Table 5: Ct Values**

| Threshold | Curvature Ct BLSD | ROC BLSD |
|---|---|---|
| -3.0 | -3.0 | Infinite |
| -1.0 | 21.3 | 2420.0 |
| -1.0 | -3.0 | Infinite |
| -1.0 | 16.6 | 947.5 |
| -1.0 | 31.6 | 1870.2 |
| -1.0 | 10.3 | 1438.8 |
| -1.0 | 28.1 | 6229.8 |
| -1.0 | 19.8 | 3216.3 |
| 29.1 | 31.7 | 6.0 |
| 29.3 | 31.6 | 5.9 |
| 29.4 | 32.0 | 6.5 |
| 29.5 | 31.7 | 6.4 |
| 29.6 | 31.6 | 5.9 |
| 29.6 | 32.1 | 5.9 |
| 29.9 | 32.0 | 6.1 |
| 30.1 | 31.8 | 6.2 |
| 30.1 | 32.0 | 6.1 |
| 30.2 | 31.9 | 6.1 |
| 30.2 | 31.8 | 6.0 |
| 30.2 | 31.8 | 5.7 |
| 30.3 | 31.7 | 5.9 |
| 30.4 | 31.9 | 6.1 |
| 30.6 | 32.1 | 5.9 |
| 30.6 | 32.2 | 6.2 |
| | | |
| **1.56%** | **0.56%** | ← ————— Cv |

Table 5 indicates that the Curvature method of calculating Ct values (in this case after normalization with BLSD) gives a smaller Cv (coefficient of variation) than the existing Threshold method. In addition, the radius of curvature (ROC) calculated with the curvature method provides a simple method of differentiating between linear curves and real growth curves.

It should be appreciated that the Ct determination processes, including the curve fitting and curvature determination processes, may be implemented in computer code running on a processor of a computer system. The code includes instructions for controlling a processor to implement various aspects and steps of the Ct determination processes. The code is typically stored on a hard disk, RAM or portable medium such as a CD, DVD, etc. Similarly, the processes may be implemented in a PCR device such as a thermocycler including a processor executing instructions stored in a memory unit coupled to the processor. Code including such instructions may be downloaded to the PCR device memory unit over a network connection or direct connection to a code source or using a portable medium as is well known.

One skilled in the art should appreciate that the elbow determination processes of the present invention can be coded using a variety of programming languages such as C, C++, C#, Fortran, VisualBasic, etc., as well as applications such as Mathematica which provide pre-packaged routines, functions and procedures useful for data visualization and analysis. Another example of the latter is MATLAB®.

**Claims**

1.  A computer-implemented method of determining the cycle threshold (Ct) value of a growth curve for a kinetic Polymerase Chain Reaction (PCR) process, comprising the steps of:

      - receiving a dataset representing a PCR growth curve, said dataset including a plurality of data points each having a pair of coordinate values (x,y), where the x-axis represents cycle number and the y-axis fluorescence intensity;

- calculating an approximation function that fits the dataset by applying a regression process to determine parameters of the function and a curve approximating the dataset;
- normalizing the curve approximating the dataset using the determined parameters to produce a normalized curve; and
- processing the normalized curve to determine a point of maximum curvature, wherein the point of maximum curvature represents the cycle threshold (Ct) value of the PCR growth curve;
wherein
- the approximation function is a double sigmoid function of the form:

$$a + bx + \frac{c}{(1 + \exp^{-d(x-e)})(1 + \exp^{-f(x-g)})}$$

- calculating includes iteratively determining the parameters a, b, c, d, e, f and g of the function by applying a Levenberg-Marquardt (LM) regression process; and
- processing includes determining the curvature at some or all points along the normalized curve.

2. The method of claim 1, further including displaying a plot of the curvature of the normalized curve.

3. The method of claim 1, wherein normalizing includes subtracting off the linear growth portion, a+bx, from the curve by applying a calculation using the formula:

$$\text{dataNew(BLS)} = \text{data-(a+bx)}$$

where dataNew(BLS) is the normalized curve after baseline subtraction, the values of parameters a and b are those values determined by the LM regression process, and x is the cycle number.

4. The method of claim 1, wherein normalizing includes dividing the curve by parameter a by applying a calculation using the formula:

$$\text{dataNew(BLD)} = \text{data/a,}$$

where dataNew(BLD) is the normalized curve after baseline division by parameter a, the value of parameter a is the value determined by the LM regression process, and x is the cycle number.

5. The method of claim 1, wherein normalizing includes subtracting off the linear growth portion, a+bx, from the curve and dividing the result by parameter a by applying a calculation using the formula:

$$\text{dataNew(BLSD)} = \text{(data-(a+bx))/a,}$$

where dataNew(BLSD) is the normalized curve after baseline subtraction with division by parameter a, the values of parameters a and b are those values determined by the LM regression process, and x is the cycle number.

6. The method of claim 1, wherein normalizing includes subtracting off the linear growth portion, a+bx, from the curve and dividing the result by parameter c by applying a calculation using the formula:

$$\text{dataNew(PGT)} = \text{(data-(a+bx))/c,}$$

where dataNew(PGT) is the normalized curve after baseline subtraction with division by parameter c, the values of parameters a, b and c are those values determined by the LM regression process, and x is the cycle number.

7. The method of claim 1, further including displaying the Ct value.

8. A computer-readable medium including code for controlling a processor to determine the cycle threshold (Ct) value

EP 1 804 172 B1

of a growth curve for a kinetic Polymerase Chain Reaction (PCR) process, the code including instructions to:

- receive a dataset representing a PCR growth curve, said dataset including a plurality of data points each having a pair of coordinate values (x,y), where the x-axis represents cycle number and the y-axis fluorescence intensity;
- calculate an approximation function that fits the dataset by applying a regression process to determine parameters of the function and a curve approximating the dataset;
- normalize the curve approximating the dataset using the determined parameters to produce a normalized curve; and
- process the normalized curve to determine a point of maximum curvature, wherein the point of maximum curvature represents the cycle threshold (Ct) value of the PCR growth curve;
wherein
- the approximation function is a double sigmoid function of the form:

$$a + bx + \frac{c}{(1 + \exp^{-d(x-e)})(1 + \exp^{-f(x-g)})};$$

- calculating includes iteratively determining the parameters a, b, c, d, e, f and g of the function by applying a Levenberg-Marquardt (LM) regression process; and
- processing includes determining the curvature at some or all points along the normalized curve.

9. The computer readable medium of claim 8, wherein the code further includes instructions to return or display the coordinate value of the point representing the cycle threshold (Ct) value of the PCR growth curve.

10. A kinetic Polymerase Chain Reaction (PCR) system, comprising:

- a kinetic PCR analysis module adapted to generate a PCR dataset representing a kinetic PCR amplification curve, said dataset including a plurality of data points, each having a pair of coordinate values (x,y), where the x-axis represents cycle number and the y-axis fluorescence intensity, wherein said dataset includes data points in a region of interest which includes a cycle threshold (Ct) value; and
- an intelligence module adapted to process the PCR dataset to determine the Ct value, by:

- calculating an approximation function that fits the dataset by applying a regression process to determine parameters of the function and a curve approximating the dataset;
- normalizing the curve approximating the dataset using the determined parameters to produce a normalized curve; and
- processing the normalized curve to determine a point of maximum curvature, wherein the point of maximum curvature represents the cycle threshold (Ct) value of the PCR growth curve;
wherein
- the approximation function is a double sigmoid function of the form:

$$a + bx + \frac{c}{(1 + \exp^{-d(x-e)})(1 + \exp^{-f(x-g)})};$$

- calculating includes iteratively determining the parameters a, b, c, d, e, f and g of the function by applying a Levenberg-Marquardt (LM) regression process; and
- processing includes determining the curvature at some or all points along the normalized curve.

11. The system of claim 10, wherein the intelligence module is further adapted to render a display of a plot of the curvature of the normalized curve.

12. The system of claim 10, wherein the kinetic PCR analysis module is resident in a kinetic thermocycler device forming part of the kinetic Polymerase Chain Reaction (PCR) system, and wherein the intelligence module includes a processor communicably coupled to the PCR analysis module.

13. The system of claim 10, wherein the intelligence module includes a processor resident in a computer system forming part of the kinetic Polymerase Chain Reaction (PCR) system coupled to the PCR analysis module by one of a network connection or a direct connection.

**Patentansprüche**

1. Computerimplementiertes Verfahren zum Bestimmen des Zyklusschwellen-(Ct)-Wertes einer Wachstumskurve für einen kinetischen Polymerasekettenreaktions-(PCR)-Prozess, umfassend die folgenden Schritte:

  - Empfangen eines Datensatzes, der eine PCR-Wachstumskurve darstellt, wobei der Datensatz eine Vielzahl von Datenpunkten einschließt, die jeweils ein Paar von Koordinatenwerten (x,y) aufweisen, wobei die x-Achse die Zykluszahl und die y-Achse die Fluoreszenzintensität darstellt;
  - Berechnen einer Näherungsfunktion, die dem Datensatz entspricht, durch Anwenden eines Regressionsprozesses zum Bestimmen von Parametern der Funktion und einer Kurvenannäherung des Datensatzes;
  - Normalisieren der Kurvenannäherung des Datensatzes unter Verwendung der bestimmten Parameter zum Erstellen einer normalisierten Kurve und
  - Verarbeiten der normalisierten Kurve zum Bestimmen eines Punktes der maximalen Krümmung, wobei der Punkt der maximalen Krümmung den Zyklusschwellen-(Ct)-Wert der PCR-Wachstumskurve darstellt;
  wobei
  - die Näherungsfunktion eine Doppelsigmoidfunktion der folgenden Form ist:

$$a + bx + \frac{c}{(1 + \exp^{-d(x-e)})(1 + \exp^{-f(x-g)})};$$

  - das Berechnen das iterative Bestimmen der Parameter a, b, c, d, e, f und g der Funktion durch Anwenden eines Levenberg-Marquardt-(LM)-Regressionsprozesses einschließt und
  - das Verarbeiten das Bestimmen der Krümmung an einigen oder allen Punkten entlang der normalisierten Kurve einschließt.

2. Verfahren nach Anspruch 1, das ferner das Anzeigen eines Plots der Krümmung der normalisierten Kurve einschließt.

3. Verfahren nach Anspruch 1, wobei das Normalisieren das Subtrahieren des linearen Wachstumsanteils, a+bx, von der Kurve durch Anwenden einer Berechnung unter Verwendung der folgenden Formel einschließt:

$$\text{DatenNeu(BLS)} = \text{Daten-(a+bx)},$$

wobei DatenNeu(BLS) die normalisierte Kurve nach Grundliniensubtraktion ist, die Werte der Parameter a und b jene Werte sind, die mit dem LM-Regressionsprozess bestimmt wurden, und x die Zykluszahl ist.

4. Verfahren nach Anspruch 1, wobei das Normalisieren das Dividieren der Kurve durch Parameter a durch Anwenden einer Berechnung unter Verwendung der folgenden Formel einschließt:

$$\text{DatenNeu(BLD)} = \text{Daten/a},$$

wobei DatenNeu(BLD) die normalisierte Kurve nach Grundliniendivision durch Parameter a ist, der Wert von Parameter a der Werte ist, der mit dem LM-Regressionsprozess bestimmt wurde, und x die Zykluszahl ist.

5. Verfahren nach Anspruch 1, wobei das Normalisieren das Subtrahieren des linearen Wachstumsanteils, a+bx, von der Kurve und das Dividieren des Ergebnisses durch Parameter a durch Anwenden einer Berechnung unter Verwendung der folgenden Formel einschließt:

$$\text{DatenNeu(BLSD)} = \text{(Daten-(a+bx))/a},$$

wobei DatenNeu(BLSD) die normalisierte Kurve nach Grundliniensubtraktion mit Division durch Parameter a ist, die Werte der Parameter a und b jene Werte sind, die mit dem LM-Regressionsprozess bestimmt wurden, und x die Zykluszahl ist.

**6.** Verfahren nach Anspruch 1, wobei das Normalisieren das Subtrahieren des linearen Wachstumsanteils, a+bx, von der Kurve und das Dividieren des Ergebnisses durch Parameter c durch Anwenden einer Berechnung unter Verwendung der folgenden Formel einschließt:

$$\mathrm{DatenNeu(PGT)} = \mathrm{(Daten\text{-}(a+bx))/c,}$$

wobei DatenNeu(PGT) die normalisierte Kurve nach Grundliniensubtraktion mit Division durch Parameter c ist, die Werte der Parameter a, b und c jene Werte sind, die mit dem LM-Regressionsprozess bestimmt wurden, und x die Zykluszahl ist.

**7.** Verfahren nach Anspruch 1, das ferner das Anzeigen des Ct-Wertes einschließt.

**8.** Computerlesbares Medium, welches einen Code zum Steuern eines Prozessors zum Bestimmen des Zyklusschwellen-(Ct)-Wertes einer Wachstumskurve für einen kinetischen Polymerasekettenreaktions-(PCR)-Prozess einschließt, wobei der Code Anweisungen einschließt, um:

- einen Datensatz zu empfangen, der eine PCR-Wachstumskurve darstellt, wobei der Datensatz eine Vielzahl von Datenpunkten einschließt, die jeweils ein Paar von Koordinatenwerten (x,y) aufweisen, wobei die x-Achse die Zykluszahl und die y-Achse die Fluoreszenzintensität darstellt;
- eine Näherungsfunktion zu berechnen, die dem Datensatz entspricht, durch Anwenden eines Regressionsprozesses zum Bestimmen von Parametern der Funktion und einer Kurvenannäherung des Datensatzes;
- die Kurvenannäherung des Datensatzes unter Verwendung der bestimmten Parameter zu normalisieren, um eine normalisierte Kurve zu erstellen; und
- die normalisierte Kurve zu verarbeiten, um einen Punkt der maximalen Krümmung zu bestimmen, wobei der Punkt der maximalen Krümmung den Zyklusschwellen-(Ct)-Wert der PCR-Wachstumskurve darstellt; wobei
- die Näherungsfunktion eine Doppelsigmoidfunktion der folgenden Form ist:

$$a + bx + \frac{c}{(1 + \exp^{-d(x-e)})(1 + \exp^{-f(x-g)})};$$

- das Berechnen das iterative Bestimmen der Parameter a, b, c, d, e, f und g der Funktion durch Anwenden eines Levenberg-Marquardt-(LM)-Regressionsprozesses einschließt und
- das Verarbeiten das Bestimmen der Krümmung an einigen oder allen Punkten entlang der normalisierten Kurve einschließt.

**9.** Computerlesbares Medium nach Anspruch 8, wobei der Code ferner Anweisungen einschließt, um den Koordinatenwert des Punktes, der den Zyklusschwellen-(Ct)-Wert der PCR-Wachstumskurve darstellt, zurückzusenden oder anzuzeigen.

**10.** System für kinetische Polymerasekettenreaktion (PCR), umfassend:

- ein Analysemodul für kinetische PCR, das dafür ausgelegt ist, einen PCR-Datensatz zu erzeugen, der eine Amplifikationskurve einer kinetischen PCR darstellt, wobei der Datensatz eine Vielzahl von Datenpunkten einschließt, die jeweils ein Paar von Koordinatenwerten (x,y) aufweisen, wobei die x-Achse die Zykluszahl und die y-Achse die Fluoreszenzintensität darstellt, wobei der Datensatz Datenpunkte in einer relevanten Region einschließt, die einen Zyklusschwellen-(Ct)-Wert einschließt, und
- ein Intelligenzmodul, das dafür ausgelegt ist, den PCR-Datensatz zu verarbeiten, um den Ct-Wert durch Folgendes zu bestimmen

- Berechnen einer Näherungsfunktion, die dem Datensatz entspricht, durch Anwenden eines Regressionsprozesses zum Bestimmen von Parametern der Funktion und einer Kurvenannäherung des Datensatzes;
- Normalisieren der Kurvenannäherung des Datensatzes unter Verwendung der bestimmten Parameter zum Erstellen einer normalisierten Kurve und

- Verarbeiten der normalisierten Kurve zum Bestimmen eines Punktes der maximalen Krümmung, wobei der Punkt der maximalen Krümmung den Zyklusschwellen-(Ct)-Wert der PCR-Wachstumskurve darstellt; wobei

- die Näherungsfunktion eine Doppelsigmoidfunktion der folgenden Form ist:

$$a + bx + \frac{c}{(1 + \exp^{-d(x-e)})(1 + \exp^{-f(x-g)})};$$

- das Berechnen das iterative Bestimmen der Parameter a, b, c, d, e, f und g der Funktion durch Anwenden eines Levenberg-Marquardt-(LM)-Regressionsprozesses einschließt und

- das Verarbeiten das Bestimmen der Krümmung an einigen oder allen Punkten entlang der normalisierten Kurve einschließt.

11. System nach Anspruch 10, wobei das Intelligenzmodul ferner dafür ausgelegt ist, eine Anzeige eines Plots der Krümmung der normalisierten Kurve wiederzugeben.

12. System nach Anspruch 10, wobei das Analysemodul für kinetische PCR in einer kinetischen Thermocycler-Vorrichtung untergebracht ist, die einen Teil des Systems für kinetische Polymerasekettenreaktion (PCR) bildet, und wobei das Intelligenzmodul einen Prozessor einschließt, der kommunikativ an das PCR-Analysemodul gekoppelt ist.

13. System nach Anspruch 10, wobei das Intelligenzmodul einen Prozessor einschließt, der in einem Computersystem untergebracht ist, das einen Teil des Systems für kinetische Polymerasekettenreaktion (PCR) bildet, das durch eines von einer Netzwerkverbindung oder einer direkten Verbindung an das PCR-Analysemodul gekoppelt ist.

**Revendications**

1. Procédé mis en œuvre par ordinateur de détermination de la valeur de cycle seuil (Ct) d'une courbe de croissance pour un procédé de réaction en chaîne par polymérase (PCR) cinétique, comprenant les étapes de :

- réception d'un jeu de données représentant une courbe de croissance par PCR, ledit jeu de données incluant une pluralité de points de données chacun ayant une paire de valeurs de coordonnées (x,y), où l'axe des x représente le nombre de cycles et l'axe des y l'intensité de fluorescence ;
- calcul d'une fonction d'approximation qui s'ajuste au jeu de données par application d'un procédé de régression pour déterminer les paramètres de la fonction et une courbe approximant le jeu de données ;
- normalisation de la courbe approximant le jeu de données en utilisant les paramètres déterminés pour produire une courbe normalisée ; et
- traitement de la courbe normalisée pour déterminer un point de courbure maximale, dans lequel le point de courbure maximale représente la valeur de cycle seuil (Ct) de la courbe de croissance par PCR ;
dans lequel
- la fonction d'approximation est une fonction double sigmoïde de la forme :

$$a + bx + \frac{c}{(1 + \exp^{-d(x-e)})(1 + \exp^{-f(x-g)})};$$

- le calcul inclut la détermination de façon itérative des paramètres a, b, c, d, e, f et g de la fonction par application d'un procédé de régression de Levenberg-Marquardt (LM) ; et
- le traitement inclut la détermination de la courbure en certains ou tous les points le long de la courbe normalisée.

2. Procédé selon la revendication 1, comprenant en outre l'affichage d'un tracé de la courbure de la courbe normalisée.

3. Procédé selon la revendication 1, dans lequel la normalisation inclut la soustraction de la partie de croissance linéaire, a+bx, de la courbe par application d'un calcul utilisant la formule :

$$dataNew(BLS) = data-(a+bx)$$

où dataNew(BLS) est la courbe normalisée après soustraction de la ligne de base, les valeurs des paramètres a et b sont les valeurs déterminées par le procédé de régression LM et x est le nombre de cycles.

4. Procédé selon la revendication 1, dans lequel la normalisation inclut la division de la courbe par le paramètre a par application d'un calcul utilisant la formule :

$$dataNew(BLD) = data/a,$$

où dataNew(BLD) est la courbe normalisée après division de la ligne de base par le paramètre a, la valeur du paramètre a est la valeur déterminée par le procédé de régression LM et x est le nombre de cycles.

5. Procédé selon la revendication 1, dans lequel la normalisation inclut la soustraction de la partie de croissance linéaire, a+bx, de la courbe et la division du résultat par le paramètre a par application d'un calcul utilisant la formule :

$$dataNew(BLSD) = (data-(a+bx))/a,$$

où dataNew(BLSD) est la courbe normalisée après soustraction de la ligne de base avec division par le paramètre a, les valeurs des paramètres a et b sont les valeurs déterminées par le procédé de régression LM et x est le nombre de cycles.

6. Procédé selon la revendication 1, dans lequel la normalisation inclut la soustraction de la partie de croissance linéaire, a+bx, de la courbe et la division du résultat par le paramètre c par application d'un calcul utilisant la formule :

$$dataNew(PGT) = (data-(a+bx))/c,$$

où dataNew(PGT) est la courbe normalisée après soustraction de la ligne de base avec division par le paramètre c, les valeurs des paramètres a, b et c sont les valeurs déterminées par le procédé de régression LM et x est le nombre de cycles.

7. Procédé selon la revendication 1, comprenant en outre l'affichage de la valeur Ct.

8. Support lisible par ordinateur incluant un code pour commander un processeur afin qu'il détermine la valeur de cycle seuil (Ct) d'une courbe de croissance pour un procédé de réaction en chaîne par polymérase (PCR) cinétique, le code incluant des instructions pour :

- la réception d'un jeu de données représentant une courbe de croissance par PCR, ledit jeu de données incluant une pluralité de points de données chacun ayant une paire de valeurs de coordonnées (x,y), où l'axe des x représente le nombre de cycles et l'axe des y l'intensité de fluorescence ;
- le calcul d'une fonction d'approximation qui s'ajuste au jeu de données par application d'un procédé de régression pour déterminer les paramètres de la fonction et une courbe approximant le jeu de données ;
- la normalisation de la courbe approximant le jeu de données en utilisant les paramètres déterminés pour produire une courbe normalisée ; et
- le traitement de la courbe normalisée pour déterminer un point de courbure maximale, dans lequel le point de courbure maximale représente la valeur de cycle seuil (Ct) de la courbe de croissance par PCR ;
dans lequel
- la fonction d'approximation est une fonction double sigmoïde de la forme :

$$a + bx + \frac{c}{(1 + \exp^{-d(x-e)})(1 + \exp^{-f(x-g)})};$$

- le calcul inclut la détermination de façon itérative des paramètres a, b, c, d, e, f et g de la fonction par application d'un procédé de régression de Levenberg-Marquardt (LM) ; et
- le traitement inclut la détermination de la courbure en certains ou tous les points le long de la courbe normalisée.

9. Support lisible par ordinateur selon la revendication 8, dans lequel le code inclut en outre des instructions pour le renvoi ou l'affichage de la valeur de coordonnée du point représentant la valeur de cycle seuil (Ct) de la courbe de croissance par PCR.

10. Système de réaction en chaîne par polymérase (PCR) cinétique, comprenant :

- un module d'analyse par PCR cinétique adapté à la génération d'un jeu de données de PCR représentant une courbe d'amplification par PCR cinétique, ledit jeu de données incluant une pluralité de points de données, chacun ayant une paire de valeurs de coordonnées (x,y), où l'axe des x représente le nombre de cycles et l'axe des y l'intensité de fluorescence, dans lequel ledit jeu de données inclut des points de données dans une région d'intérêt qui inclut une valeur de cycle seuil (Ct) ; et
- un module d'intelligence adapté au traitement du jeu de données de PCR afin de déterminer la valeur Ct, par :

- le calcul d'une fonction d'approximation qui s'ajuste au jeu de données par application d'un procédé de régression pour déterminer les paramètres de la fonction et une courbe approximant le jeu de données ;
- la normalisation de la courbe approximant le jeu de données en utilisant les paramètres déterminés pour produire une courbe normalisée ; et
- le traitement de la courbe normalisée pour déterminer un point de courbure maximale, dans lequel le point de courbure maximale représente la valeur de cycle seuil (Ct) de la courbe de croissance par PCR ;

dans lequel
- la fonction d'approximation est une fonction double sigmoïde de la forme :

$$a + bx + \frac{c}{(1 + \exp^{-d(x-e)})(1 + \exp^{-f(x-g)})};$$

- le calcul inclut la détermination de façon itérative des paramètres a, b, c, d, e, f et g de la fonction par application d'un procédé de régression de Levenberg-Marquardt (LM) ; et
- le traitement inclut la détermination de la courbure en certains ou tous les points le long de la courbe normalisée.

11. Système selon la revendication 10, dans lequel le module d'intelligence est en outre adapté à la restitution d'un affichage du tracé de la courbure de la courbe normalisée.

12. Système selon la revendication 10, dans lequel le module d'analyse par PCR cinétique réside dans un dispositif thermocycleur cinétique formant une partie du système de réaction en chaîne par polymérase (PCR) cinétique, et dans lequel le module d'intelligence inclut un processeur couplé de manière communicante au module d'analyse par PCR.

13. Système selon la revendication 10, dans lequel le module d'intelligence inclut un processeur résidant dans un système informatique formant une partie du système de réaction en chaîne par polymérase (PCR) cinétique couplé au module d'analyse par PCR au moyen d'une parmi une connexion réseau ou une connexion directe.

**FIG. 1**

**FIG. 2**

EP 1 804 172 B1

# FIG. 3

## Spike identification and replacement process flowchart

This flowchart models the Levenberg-Marquardt outlier method
File: LMOM_SpikeDetector.cs
Class: LMOM_SpikeDetector
Function: CorrectSpikes
Lines: 256-555

The equation used to model PCR curves is a double sigmoid:
$a + bx + c / (1+Exp(-d*(x-e))) (1+Exp(-f*(x-g)))$

Variables Summary

a: 1st parameter of the double sigmoid equation
b: 2nd parameter of the double sigmoid equation
c: 3rd parameter of the double sigmoid equation
d: 4th parameter of the double sigmoid equation
e: 5th parameter of the double sigmoid equation
f: 6th parameter of the double sigmoid equation
g: 7th parameter of the double sigmoid equation
MAPETreshold: Cutoff for the MAPE value of a curve fit to be considered valid
ziTreshold1: Cutoff value for the Z-Value of a point to be considered a spike
ziTreshold2: Cutoff value for the Z-Value of a point to be considered a big spike

**502**

Set Parameters b,d and f.

Do/ Set (d,f) = (0.1,0.7), (1.0,0.4), (0.35,0.25)
Do/ Set b =0.01 for all inital parameter sets.

These parameters don't depend on the curve.

**504**

Set Parameters a and c

Do/ Set a = 3rd lowest Y-Value for every set of initial parameters.
Do/ Set c = 3rd highest Y-Value -a for the two first sets of initial parameters.
Do/ Set c = 3rd highest Y-Value-a+2 for the last set of initial parameters.

These parameters depend on the curve and require minimum calculation.

Set Parameters e and g

**506**

These parameters depend on the curve and require more calculation.

**510**

Set the LM with the initial set of parameters.

23

FIG. 3 (cont.)

Run Levenberg-Marquardt

■ Exit/ The LM Returns the fit value of its approximation

This time, the curve doesn't
contain any big spikes any more.
The result should therefore be
more accurate                    ● ●

[The fit is worse than the best fit]

[The fit is better than the best fit]
                    Save the best Parameters

    ■ Do/ Save the best fit value
    ■ Do/ Save the best set of parameters

[All initial parameters have been tried]

                [There are initial parameters that
                          haven't been tried]

                    Set the LM with the next
                    set of parameters

                    Get Z-Values
                              ● ●

            Count and record the small spike points

    ■ Do/ For every point above the ziTreshold1: record its position
    ■ Do/ For every point above the ziTreshold1: spikePoints++

| Return a bad approximation error code | Replace spike points with cubic spline approximation | The Curve contains no spikes. |
|---|---|---|
| ■ Do/ Create a return array result curve of length 1<br>■ Do/ Fill both return arrays with -1 error code<br>■ Do/Create a return array spikePoints of length 1<br>■ Exit/ Return the result curve and spikePoints arrays | ■ Exit/ Returns a corrected curve as result curve<br>■ Exit/ Returns an array of the positions of the corrected spikes<br>                    ● ● | ■ Exit/ Return a hard copy of the curve as result curve<br>■ Exit/ Return an empty array as SpikePoints |

**FIG. 3 (cont.)**

## FIG. 4

**Double sigmoid decomposition**

a constant term
b linear term
s1 sigmoid 1
s2 sigmoid 2
s resultant double sigmoid

## FIG. 5

**The parameters of a sigmoid**

**FIG. 6**

Initial parameters shapes

# FIG. 7

## Parameter e and g calculation flowchart

This flowchart models the way the double-sigmoid parameters e and g are calculated
File: LMOMSpike_Detecor.cs
Class: LMOM_SpikeDetector
Function: CorrectSpikes
Lines: 302-340

Variables Summary:

e: current value of the parameter e
g: current value of the parameter g
mean: value of the mean of the y-values of the curve
numCycles: the number of cycles of the curve
toCorrect: an array containing the y-values of the curve
preSpikeLoops: stores how many candidates for e and g have been tested

Get the mean of the
fluorescence.

■Do/ mean = Mean(toCorrect)

Set e = 5

[e > numCycles - 5]

[e < numCycles - 5]

[toCorrect[e]<mean]

↓[toCorrect[e] > mean]
Increase the value of the
Spike Checking loops by
one.

■Do/ preSpikeLoops++

[The Derivatives around e don't have
sign change]

[The derivatives around e have a sign
change]

Increase e

■Do/e++

Return e = g = 2/3
Number of Cycles

■Do/ e=g=2/3*numCycles

Return e = g = e - the
number of Spike Checking
loops .

■Do/e=g=e-preSpikeLoops

The reason we substract the
number of loops that were
candidates, is that if the curve has
a lot of noise, it will still give us a
good approximate value.

## FIG. 8

### Levenberg-Marquardt Process flowchart

This flowchart models the Levenberg-Marquardt algorithm
File: LevenMarNR.cs
Class: LevenMarNR
Function: CalculateParams
Lines: 69-215

Variables Summary

da: vector containing the increment for the new parameter set
diff: the difference between the sum of absolute errors of two consecutive iterations
iteration: the number of iteration done
lamda: the damping factor of the Levenberg-Marquardt method
maxIteration: the maximum number of iteration
oneda: Jacobian^T*Error
temp: the Hessian Matrix, augmented during the "Augment the Hessian Matrix" activity

Set the initial variables

Do/ diff = 2.0
Do/ iteration = 0
Do/ lamda = 0.001

Evaluate the fit of a set of
parameters and prepare
matrices

The parameters used for this first
iterations are the initial
parameters previously loaded.

The Matrices prepared during this
step are the Hessian Matrix of the
fit and the Jacobian Matrix *
(y[pred]-y[data])

[iterations>maxIterations || diff <
0.0001 || lamda > 10^20]

maxIteration default value is 100.

[iterations<maxIterations && diff >
0.0001 && lamda < 10^20]

Increment iteration

Do/iteration++

Augment the Hessian Matrix

Do/ Add lamda to the diagonal of temp

Solve the augmented equation:
temp * oneda = da

■ Exit/ Returns da, the solution vector

da is the Jacobian Matrix*(y[pred]-y[data])

■ \Error during Gauss-Jordan\

temp is the augmented Hessian Matrix.

The equation is solved using the
Gauss-Jordan Method

Increment Parameters

■ Do/ Add da to the current parameters

The previous set of parameters is
stored in a different array.

Evaluate the fit of a set
of parameters and
prepare matrices

[newFit < oldFit && -1 < parameter        [newFit > oldFit || -1 > parameter d,f
d,f < 1.1]        || parameter d,f > 1.1]

Test wether the new set of
parameters fits the data better.

Keep new set of parameters

■ Do/ Decrease lamda: lamda = 0.1*lamda
■ Do/ update the best fit value
■ Do/ update the best set of parameters
■ Do/ update the difference between two iterations, diff = oldFit-newFit

Throw away new set of
parameters

■ Do/ Increase lamda: lamda = 10*lamda

Return the current set of parameters

■ Exit/ The last set of parameters is returned
■ Exit/ The value of the absolute sum of errors of the best fit is returned

Return the best parameters so far

■ Exit/ Its fit in terms of sum of absolute error is returned
■ Exit/ The best set of parameters calculated before the error is returned

**FIG. 8 (cont.)**

**FIG. 9**

```
┌─────────────────────────────┐
│      Acquire Data set       │──── 910
└─────────────────────────────┘
               │
               ▼
┌─────────────────────────────┐
│     Approximate Curve Fit   │──── 920
└─────────────────────────────┘
               │
               ▼
┌─────────────────────────────┐
│       Normalize Data        │──── 930
└─────────────────────────────┘
               │
               ▼
┌─────────────────────────────┐
│       Apply Curvature       │
│  Determination Process to   │──── 940
│    Determine Elbow Value    │
└─────────────────────────────┘
               │
               ▼
┌─────────────────────────────┐
│      Return Elbow value     │──── 950
│                             │
└─────────────────────────────┘
               │
               ▼
┌─────────────────────────────┐
│        Display Data         │──── 960
└─────────────────────────────┘
```

## FIG. 10

### FIG. 10a: PCR data

### FIG. 10b: Curvature

## FIG. 11

### Radius of Curvature

Radius =

**FIG 12a**

| Cycle Number | Signal | Cycle Number | Signal |
|---|---|---|---|
| 1 | 1,47 | 31 | 1,82 |
| 2 | 1,52 | 32 | 1,83 |
| 3 | 1,52 | 33 | 1,86 |
| 4 | 1,53 | 34 | 2,05 |
| 5 | 1,52 | 35 | 2,47 |
| 6 | 1,51 | 36 | 2,94 |
| 7 | 1,54 | 37 | 3,43 |
| 8 | 1,52 | 38 | 3,93 |
| 9 | 1,55 | 39 | 4,32 |
| 10 | 1,51 | 40 | 4,69 |
| 11 | 1,60 | 41 | 4,94 |
| 12 | 1,57 | 42 | 5,27 |
| 13 | 1,60 | 43 | 5,59 |
| 14 | 1,59 | 44 | 5,88 |
| 15 | 1,57 | 45 | 6,12 |
| 16 | 1,64 | 46 | 6,43 |
| 17 | 1,63 | 47 | 6,78 |
| 18 | 1,63 | 48 | 7,08 |
| 19 | 1,68 | 49 | 7,35 |
| 20 | 1,60 | 50 | 7,67 |
| 21 | 1,69 | 51 | 7,92 |
| 22 | 1,70 | 52 | 8,21 |
| 23 | 1,63 | 53 | 8,54 |
| 24 | 1,76 | 54 | 8,86 |
| 25 | 1,71 | 55 | 9,11 |
| 26 | 1,74 | 56 | 9,36 |
| 27 | 1,70 | 57 | 9,65 |
| 28 | 1,68 | 58 | 9,89 |
| 29 | 1,81 | 59 | 10,15 |
| 30 | 1,81 | 60 | 10,44 |

**FIG. 12b**

**Raw Data**

**FIG. 13**

**Raw Data and Double Sigmoid Fit**

**FIG. 14**

**Normalized Data and Double Sigmoid Fit**

**FIG. 15**

**Curvature Plot**

**FIG. 16**

Radius of Curvature

**FIG. 17**

Raw Data

**FIG. 18**

Baseline Subtraction and Double Sigmoid

## FIG. 19

**Curvature Plot**

## FIG. 20

**Raw Data Set**

replicate

negative

**FIG. 21**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9746707 A
- WO 9746712 A
- WO 9746714 A
- US 2003148302 A
- EP 1647910 A

### Non-patent literature cited in the description

- **GIBSON et al.** *Genome Research,* vol. 6, 995-1001
- **BIECHE et al.** *Cancer Research,* 1999, vol. 59, 2759-2765
- **TICHOPAD et al.** *Biotechnology Letters,* 2002, vol. 24, 2053-2056
- **WANG, SHA-SHA et al.** Homogeneous Real-Time Detection of Single-Nucleotide Polymorphisms by Strand Displacement Amp lification on the BD ProbeTec ET System. *Clin Chem,* 2003, vol. 49 (10), 1599
- **WEUSTEN, JOS J.A.M. et al.** Principles of Quantitation of Viral Loads Using Nucleic Acid Sequence-Based Amplification in Combination With Homogeneous Detection Using Molecular Beacons. *Nucleic Acids Research,* 2002, vol. 30 (6), 26